(19) Europäisches Patentamt
European Patent Office
Office européen des brevets

(11) EP 4 318 701 A1

(12) **EUROPEAN PATENT APPLICATION**
published in accordance with Art. 153(4) EPC

(43) Date of publication:
**07.02.2024 Bulletin 2024/06**

(21) Application number: **21966717.7**

(22) Date of filing: **08.12.2021**

(51) International Patent Classification (IPC):
***H01M 10/052*** *(2010.01)*

(52) Cooperative Patent Classification (CPC):
**H01M 10/052;** Y02E 60/10

(86) International application number:
**PCT/CN2021/136599**

(87) International publication number:
**WO 2023/102793 (15.06.2023 Gazette 2023/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Contemporary Amperex Technology Co., Limited**
**Ningde, Fujian 352100 (CN)**

(72) Inventors:
- **ZOU, Hailin**
**Ningde, Fujian 352100 (CN)**
- **CHEN, Peipei**
**Ningde, Fujian 352100 (CN)**

(74) Representative: **Gong, Jinping**
**CocreateIP**
**Neumarkterstr. 21**
**81673 München (DE)**

(54) **SECONDARY BATTERY AND ELECTRICAL APPARATUS COMPRISING SAME**

(57) The present application provides a secondary battery and an electrical apparatus containing the same. The secondary battery includes a positive electrode sheet, a negative electrode sheet, a separator disposed between the positive electrode sheet and the negative electrode sheet, and an electrolyte solution, wherein the separator includes a substrate and an inorganic and/or organic particle layer disposed on at least one surface of the substrate, and the separator further includes an acid scavenging additive located among the inorganic and/or organic particles of the inorganic and/or organic particle layer or located in a form of a separate layer between the substrate and the inorganic and/or organic particle layer, wherein the maximum dissolution amount of the acid scavenging additive in the electrolyte solution at 25°C is less than 1% by mass relative to the mass of the electrolyte solution.

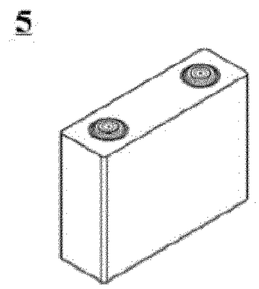


FIG. 1

EP 4 318 701 A1

## Description

### Technical Field

The present application relates to the technical field of secondary batteries, and in particular to a secondary battery and an electrical apparatus containing the same.

### Background Art

Secondary batteries are widely applied due to their outstanding features such as high energy density, no pollution, and long service life. However, in the secondary battery, an electrolyte solution is sensitive to moisture, so it is easy to generate acidic substances, thereby destructing the performance of the battery. Specifically, the acidic substances are not resistant to electrochemical oxidation and reduction, so they will consume active lithium in the battery, reduce coulombic efficiency, deteriorate internal resistance of the battery, reduce battery capacity and increase gas evolution, etc.; and acidic substances will also etch an SEI film, so that an interface between positive and negative electrodes are destructed continuously and transition metals in the electrodes are dissolved continuously, which eventually leads to problems such as increased gas evolution, deterioration of cycling performance and storage performance, etc.

Currently, a conventional technical means is to add a moisture or acid scavenging additive into the electrolyte solution to inhibit the destruction of the battery performance by the acidic substances. For example, patent documents CN105006594B and CN105633467B report use of a P- or N-containing additive such as tris(trimethylsilane)phosphite, N,N-dimethylpropionamide, N-methyl-2-pyrrolidone and N-substituted pyrrolidone-boron trifluoride coordination compounds, etc. A small amount of such additives added into the electrolyte solution shows a weak Lewis basicity, so that it can form a hexa-coordinated complex with $PF_5$, so as to reduce the Lewis acidity and reactivity of $PF_5$ and inhibit the increase in chromaticity and the like caused by a reaction between $PF_5$ and a trace amount of impurities in the electrolyte solution. Furthermore, a method of adding a cyclic anhydride compound into the electrolyte solution reported in patent document CN110970621B and a method of adding thiocyanate and isothiocyanate additives into the electrolyte solution reported in patent document CN1194439C can also reduce the acidity of the electrolyte solution. However, the products formed by the reaction of these additives with acidic substances and water are still electrochemically active, so an electrochemical side reaction will occur at the positive and negative electrodes, and eventually lead to deterioration of battery performance, such as increased battery expansion and reduced coulombic efficiency. Moreover, these additives themselves also continue to react at the surface of the positive and negative electrodes, so that they are depleted in an initial cycle of the battery and thus cannot exist in the battery for a long time. Therefore, these additives cannot achieve the effect of acid scavenging during the long-term cycle of the battery. CN102709591B reported a hydrophobic separator, in which the moisture introduced by the battery manufacturing process can be reduced by coating long-chain alcohol, ketone, aldehyde, ester, ether and alkane polymers with hydrophobic properties on a substrate. However, this method cannot change the content of acidic substances and water in the prepared battery, and has no scavenging effect on the acidic substances formed during the solvent oxidation process.

### Summary of the Invention

The present application has been made in view of the aforementioned problems, and an objective of the present application is to provide a secondary battery that can control the acid content in a battery system to a relatively low level for a long period of time due to inclusion of a specific separator, thereby reducing side reactions in the battery and consumption of active lithium and thus significantly improving the cycling performance and storage performance, especially the high temperature cycling performance and high temperature storage performance of the battery. Further, an objective of the present application is also to provide a battery module, battery pack and electrical apparatus including the secondary battery.

In order to achieve the aforementioned objectives, a first aspect of the present application provides a secondary battery, which includes a positive electrode sheet, a negative electrode sheet, a separator disposed between the positive electrode sheet and the negative electrode sheet, and an electrolyte solution, wherein the separator includes a substrate and an inorganic and/or organic particle layer disposed on at least one surface of the substrate, and the separator further includes an acid scavenging additive located among the inorganic and/or organic particles of the inorganic and/or organic particle layer or located in a form of a separate layer between the substrate and the inorganic and/or organic particle layer, wherein the maximum dissolution amount of the acid scavenging additive in the electrolyte solution at 25°C is less than 1% by mass relative to the mass of the electrolyte solution.

In some embodiments, the acid scavenging additive includes at least one selected from a pyridine compound and a phosphine compound,

the pyridine compound is at least one selected from compounds of chemical formulas I, II and III:

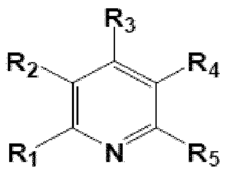

chemical formula I

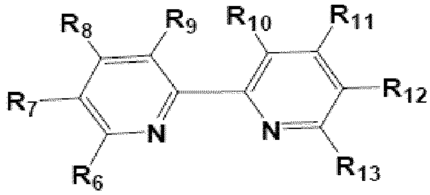

chemical formula II

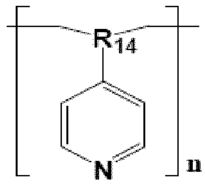

chemical formula III

the phosphine compound is at least one selected from compounds of chemical formula IV:

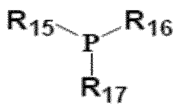

chemical formula IV

wherein $R_1$ to $R_{13}$ and $R_{15}$ to $R_{17}$ are each independently one selected from the following: alkyl having 1-20 carbon atoms, alkenyl having 2-20 carbon atoms, aryl having 6-26 carbon atoms, aryloxy having 6-26 carbon atoms, and substituted groups thereof formed by substitution with F, Cl, Br, a sulfonic group or sulfonyl, and H, F, Cl and Br; $R_{14}$ is one selected from the following: alkylene having 1-20 carbon atoms, alkenylene having 2-20 carbon atoms, arylene having 7-26 carbon atoms, aryleneoxy having 7-26 carbon atoms, and substituted groups thereof formed by substitution with F, Cl, Br, a sulfonic group or sulfonyl, and n is an integer greater than or equal to 2.

In some embodiments, the pyridine compound includes at least one selected from an isonicotinate, 2-hydroxypyridine-4-carboxylate, 2,2'-bipyridine-4,4'-dicarboxylate, and poly(4-vinylpyridine).

In some embodiments, the isonicotinate includes at least one selected from lithium isonicotinate, sodium isonicotinate, potassium isonicotinate, aluminum isonicotinate, magnesium isonicotinate, calcium isonicotinate, iron isonicotinate, cobalt isonicotinate, nickel isonicotinate and manganese isonicotinate;

The 2-hydroxypyridine-4-carboxylate includes at least one selected from lithium 2-hydroxypyridine-4-carboxylate, sodium 2-hydroxypyridine-4-carboxylate, potassium 2-hydroxypyridine-4-carboxylate, aluminum 2-hydroxypyridine-4-carboxylate, magnesium 2-hydroxypyridine-4-carboxylate, calcium 2-hydroxypyridine-4-carboxylate, iron 2-hydroxypyridine-4-carboxylate, cobalt 2-hydroxypyridine-4-carboxylate, nickel 2-hydroxypyridine-4-carboxylate, and manganese 2-hydroxypyridine-4-carboxylate; and

the 2,2'-bipyridine-4,4'-dicarboxylate includes at least one selected from sodium 2,2'-bipyridine-4,4'-dicarboxylate, potassium 2,2'-bipyridine-4,4'-dicarboxylate, aluminum 2,2'-bipyridine-4,4'-dicarboxylate, magnesium 2,2'-bipyridine-4,4'-dicarboxylate, calcium 2,2'-bipyridine-4,4'-dicarboxylate, iron 2,2'-bipyridine-4,4'-dicarboxylate, cobalt 2,2'-bipyridine-4,4'-dicarboxylate, nickel 2,2'-bipyridine-4,4'-dicarboxylate and manganese 2,2'-bipyridine-4,4'-dicarboxylate.

In some embodiments, the phosphine compound includes at least one selected from diethylphenylphosphine, trihexylphosphine, tributylphosphine, 1,6-bis(diphenylphosphino)hexane, tris(o-methoxyphenyl)phosphine, tri-n-octylphosphine, diphenylcyclohexylphosphine, diphenyl-2-pyridinephosphine, 2-(dicyclohexylphosphino)biphenyl, 2-(di-tert-butylphosphino)biphenyl, diphenylethoxyphosphine, tris(2-furyl)phosphine, tricyclopentylphosphine, and diethylphenylphosphine.

In some embodiments, the loading amount of the acid scavenging additive on the separator is 0.01 $g/m^2$-20 $g/m^2$, and optionally 0.1 $g/m^2$-5 $g/m^2$.

In some embodiments, the thickness of the inorganic and/or organic particle layer is 0.1 $\mu m$-10 $\mu m$, and optionally 0.5 $\mu m$-5 $\mu m$.

In some embodiments, the inorganic particle includes at least one selected from the group consisting of silica, alumina, boehmite, magnesia, titanium dioxide, zinc oxide, and magnesium aluminate oxide; the organic particle includes at least one selected from the group consisting of polyethylene oxide particles, polyvinylidene chloride particles, polyacrylonitrile particles, polyvinylidene fluoride particles, polymethyl methacrylate particles, and poly(vinylidene fluoride-hexafluoropropylene) copolymer particles.

In some embodiments, the electrolyte solution includes a solvent and an electrolyte salt, and optionally, the solvent includes at least one selected from a carbonate solvent, a sulfone solvent, an ether solvent, a sulfonate solvent, a sulfate solvent and a sulfite solvent; and the electrolyte salt includes a lithium or sodium salt.

In some embodiments, the substrate includes at least one selected from polyethylene, polypropylene, cellulose and polyimide.

A second aspect of the present application provides a battery module including the secondary battery provided by the first aspect of the present application.

A third aspect of the present application provides a battery pack including the battery module provided by the second aspect of the present application.

A fourth aspect of the present application provides an electrical apparatus including at least one selected from the secondary battery according to the first aspect of the present application, the battery module according to the second aspect of the present application, or the battery pack according to the third aspect of the present application.

Beneficial effects

The present application provides a secondary battery in which an acid scavenging additive having low solubility in an electrolyte solution is introduced on a separator and the acid scavenging additive is disposed among the inorganic and/or organic particles of the inorganic and/or organic particle layer, or the acid scavenging additive is disposed in a form of a separate layer between the substrate and the inorganic and/or organic particle layer, so that the acid content in the battery system can be controlled to a relatively low level for a long period of time, thereby reducing side reactions in the battery and consumption of active lithium and thus significantly improving the cycling performance and storage performance, especially the high temperature cycling performance and high temperature storage performance of the battery.

The battery module, battery pack, and electrical apparatus of the present application includes the secondary battery provided by the present application, and thus have at least the same advantages as those of the secondary battery.

## Description of Drawings

In order to illustrate the technical solutions of the examples of the present application more clearly, the drawings required in the examples of the present application will be briefly introduced hereafter. Obviously, the drawings described hereafter are only some examples of the present application, and they can be modified by those of ordinary skills in the art according to the teaching of the present application without any creative effort.

Fig. 1 is a schematic view of a secondary battery according to an embodiment of the present application.

Fig. 2 is an exploded view of the secondary battery according to the embodiment of the present application shown in Fig. 1.

Fig. 3 is a schematic view of a battery module according to an embodiment of the present application.

Fig. 4 is a schematic view of a battery pack according to an embodiment of the present application.

Fig. 5 is an exploded view of the battery pack according to the embodiment of the present application shown in Fig. 4.

Fig. 6 is a schematic view of an electrical apparatus in which a secondary battery is used a power source according to an embodiment of the present application.

Description of reference numerals:

1 battery pack; 2 upper box; 3 lower box; 4 battery module; 5 lithium ion battery; 51 case; 52 electrode assembly; 53 top cover assembly.

## Detailed Description

Hereinafter, the embodiments of the secondary battery, battery module, battery pack and electrical apparatus of the present application are specifically disclosed by referring to the detailed description of the drawings as appropriate. However, there may be cases where unnecessary detailed description is omitted. For example, there are cases where detailed descriptions of well-known items and repeated descriptions of actually identical structures are omitted. This is to avoid unnecessary redundancy in the following descriptions and to facilitate the understanding by those skilled in the art. In addition, the drawings and subsequent descriptions are provided for those skilled in the art to fully understand the present application, and are not intended to limit the subject matter recited in the claims.

A "range" disclosed in the present application is defined in the form of a lower limit and an upper limit, with a given range being defined by the selection of a lower limit and an upper limit, and the selected lower and upper limits defining the boundaries of the particular range. A range defined in this manner may be inclusive or exclusive of end values, and may be arbitrarily combined, that is, any lower limit may be combined with any upper limit to form a range. For example, if ranges of 60-120 and 80-110 are listed for a particular parameter, it is understood that ranges of 60-110

and 80-120 are also contemplated. Additionally, if the minimum range values 1 and 2 are listed, and if the maximum range values 3, 4, and 5 are listed, the following ranges are all contemplated: 1-3, 1-4, 1-5, 2- 3, 2-4 and 2-5. In the present application, unless stated otherwise, the numerical range "a-b" represents an abbreviated representation of any combination of real numbers between a and b, where both a and b are real numbers. For example, the numerical range "0-5" means that all real numbers between "0-5" have been listed herein, and "0-5" is just an abbreviated representation of the combination of these numerical values. Additionally, when it is stated that a certain parameter is an integer of ≥ 2, it is equivalent to disclosing that the parameter is, for example, an integer of 2, 3, 4, 5, 6, 7, 8, 9, 10, 11, 12, etc.

Unless otherwise specified, all embodiments and optional embodiments of the present application may be combined with each other to form new technical solutions.

Unless otherwise specified, all technical features and optional technical features of the present application may be combined with each other to form new technical solutions.

Unless otherwise specified, all steps of the present application may be performed sequentially or randomly, and preferably sequentially. For example, the method includes steps (a) and (b), meaning that the method may include steps (a) and (b) performed sequentially, or may include steps (b) and (a) performed sequentially. For example, the reference to the method may further include step (c), meaning that step (c) may be added to the method in any order. For example, the method may include steps (a), (b) and (c), or may further include steps (a), (c) and (b), or may further include steps (c), (a) and (b), and the like.

In the description herein, it should be noted that, unless otherwise specified, "or more" and "or less" encompass the recited number itself, and "more" in "one or more" means two or more.

Embodiments of the present application will be described in detail hereinafter. [secondary battery]

A first embodiment of the present application can provide a secondary battery, which includes a positive electrode sheet, a negative electrode sheet, a separator disposed between the positive electrode sheet and the negative electrode sheet, and an electrolyte solution, wherein the separator includes a substrate and an inorganic and/or organic particle layer disposed on at least one surface of the substrate, and the separator further includes an acid scavenging additive located among the inorganic and/or organic particles of the inorganic and/or organic particle layer or located in a form of a separate layer between the substrate and the inorganic and/or organic particle layer, wherein the maximum dissolution amount of the acid scavenging additive in the electrolyte solution at 25°C is less than 1% by mass relative to the mass of the electrolyte solution. In the present application, "the maximum dissolution amount of acid scavenging additive in the electrolyte solution at 25°C" refers to the mass ratio of the acid scavenging additive dissolved in the electrolyte solution at 25°C relative to the sum of the acid scavenging additive and the electrolyte solution.

In the present application, the term "inorganic and/or organic particle" refers to an inorganic particle alone, an organic particle alone, or a mixture of both. For example, the term "inorganic and/or organic particle layer" refers to a layer of inorganic particles alone, a layer of organic particles alone, or a mixture of both. Specific examples will be described in detail in the Examples section hereafter.

In the secondary battery of the present application, the acid scavenging additive is disposed on the separator. During a charging and discharging process, acidic substances in the electrolyte solution reacts with the acid scavenging additive on the separator or forms a complex with the acid scavenging additive, so that the acid content in the battery system can be controlled at a relatively low level. Furthermore, in the present application, the acid scavenging additive is disposed among the inorganic and/or organic particles of the inorganic and/or organic particle layer or disposed in the form of a separate layer between the substrate and the inorganic and/or organic particle layer, and has low solubility in the electrolyte solution (i.e., the acid scavenging additive is insoluble or slightly soluble in the electrolyte solution). Therefore, the acid scavenging additive cannot be in direct contact with positive and negative electrodes, so that there is almost no electrochemical redox reaction between them. This ensures that the acid scavenging additive is present in the battery system throughout the life cycle of the battery. Moreover, compared with the acidic substances, the size of the product obtained after the aforementioned acid scavenging additive reacts with or is coordinated with the acidic substances is larger, so it is not easy for the product to diffuse into the electrolyte solution through the inorganic and/or organic particle layer. Therefore, even in the case where the aforementioned product can undergo an electrochemical reaction at the positive and negative electrodes, since the aforementioned product cannot be in direct contact with the positive and negative electrodes, there is almost no electrochemical redox reaction between them. With the aforementioned configuration, the acid content in the battery system can be controlled to a relatively low level for a long period of time, thereby reducing side reactions in the battery and consumption of active lithium and thus significantly improving the cycling performance and storage performance, especially the high temperature cycling performance and high temperature storage performance of the battery.

In some embodiments, the acid scavenging additive can include at least one selected from a pyridine compound and a phosphine compound,

the pyridine compound is at least one selected from compounds of chemical formulas I, II and III:

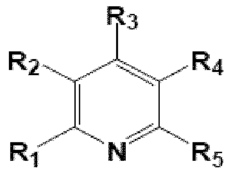

chemical formula I

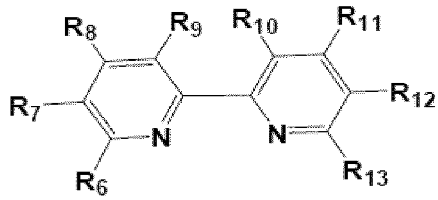

chemical formula II

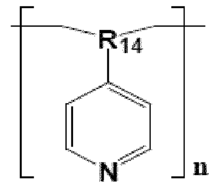

chemical formula III

the phosphine compound can be at least one selected from compounds of chemical formula IV:

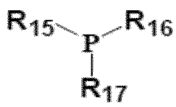

chemical formula IV

wherein $R_1$ to $R_{13}$ and $R_{15}$ to $R_{17}$ are each independently one selected from the following: alkyl having 1-20 carbon atoms, alkenyl having 2-20 carbon atoms, aryl having 6-26 carbon atoms, aryloxy having 6-26 carbon atoms, and substituted groups thereof formed by substitution with F, Cl, Br, a sulfonic group or sulfonyl, and H, F, Cl and Br; $R_{14}$ is one selected from the following: alkylene having 1-20 carbon atoms, alkenylene having 2-20 carbon atoms, arylene having 7-26 carbon atoms, aryleneoxy having 7-26 carbon atoms, and substituted groups thereof formed by substitution with F, Cl, Br, a sulfonic group or sulfonyl, and n is an integer greater than or equal to 2.

The N atom on the aforementioned pyridine compound and the P atom on the phosphine compound have a lone electron pair, so these compounds have Lewis basicity and can react with the acidic substances to remove the acidic substances, or form a complex with the acidic substances to fix the acidic substances in the separator. As a result, the content of the acidic substances in the electrolyte solution can be reduced, so that the acidic substances in the battery system cannot react at an interface of the positive and negative electrodes, thereby reducing side reactions and consumption of active lithium in the battery for a long period of time. Therefore, the cycling performance and storage performance, especially the high temperature cycling performance and high temperature storage performance of the battery can be significantly improved.

In some embodiments, from the viewpoint of improving the cycling performance and storage performance, especially the high temperature cycling performance and high temperature storage performance of the battery, the pyridine compound can include at least one selected from an isonicotinate, 2-hydroxypyridine-4-carboxylate, 2,2'-bipyridine-4,4'-dicarboxylate, and poly(4-vinylpyridine). In the present application, there is no particular limitation on the weight-average molecular weight of poly(4-vinylpyridine), as long as poly(4-vinylpyridine) is slightly soluble or insoluble in the electrolyte solution and can achieve the acid scavenging effect, and for example, the weight-average molecular weight may be 40,000 to 900,000. Optionally, the weight-average molecular weight of poly(4-vinylpyridine) may be 50,000 to 850,000, 60,000 to 800,000, 70,000 to 750,000, 75,000 to 700,000, 80,000 to 650,000, 100,000 to 550,000, 150,000 to 450,000, 200,000 to 400,000, 250,000 to 350,000, 300,000 to 350,000, and 320,000 to 550,000.

In some embodiments, there is no particular limitation on the cation of the pyridine-containing carboxylate, and a cation commonly used in electrolyte solution can be used. From the viewpoint of improving the cycling performance and storage performance, especially the high temperature cycling performance and high temperature storage performance of the battery, the isonicotinate can include at least one selected from lithium isonicotinate, sodium isonicotinate, potassium isonicotinate, aluminum isonicotinate, magnesium isonicotinate, calcium isonicotinate, iron isonicotinate, cobalt isonicotinate, nickel isonicotinate and manganese isonicotinate; the 2-hydroxypyridine-4-carboxylate can include at least one selected from lithium 2-hydroxypyridine-4-carboxylate, sodium 2-hydroxypyridine-4-carboxylate, potassium 2-hydroxypyridine-4-carboxylate, aluminum 2-hydroxypyridine-4-carboxylate, magnesium 2-hydroxypyridine-4-carboxylate, calcium 2-hydroxypyridine-4-carboxylate, iron 2-hydroxypyridine-4-carboxylate, cobalt 2-hydroxypyridine-4-carboxylate, nickel 2-hydroxypyridine-4-carboxylate, and manganese 2-hydroxypyridine-4-carboxylate; and the 2,2'-bipyridine-4,4'-dicarboxylate can include at least one selected from sodium 2,2'-bipyridine-4,4'-dicarboxylate, potassium 2,2'-bipyridine-4,4'-dicarboxylate, aluminum 2,2'-bipyridine-4,4'-dicarboxylate, magnesium 2,2'-bipyridine-4,4'-dicarboxylate, calcium 2,2'-bipyridine-4,4'-dicarboxylate, iron 2,2'-bipyridine-4,4'-dicarboxylate, cobalt 2,2'-bipyridine-4,4'-dicarboxylate, nickel 2,2'-bipyridine-4,4'-dicarboxylate and manganese 2,2'-bipyridine-4,4'-dicarboxylate.

In some embodiments, from the viewpoint of improving the cycling performance and storage performance, especially the high temperature cycling performance and high temperature storage performance of the battery, the phosphine compound can include at least one selected from diethylphenylphosphine, trihexylphosphine, tributylphosphine, 1,6-bis(diphenylphosphino)hexane, tris(o-methoxyphenyl)phosphine, tri-n-octylphosphine, diphenylcyclohexylphosphine, diphenyl-2-pyridinephosphine, 2-(dicyclohexylphosphino)biphenyl, 2-(di-tert-butylphosphino)biphenyl, diphenylethoxyphosphine, tris(2-furyl)phosphine, tricyclopentylphosphine, and diethylphenylphosphine.

In some embodiments, the loading amount of the acid scavenging additive on the separator can be 0.01 $g/m^2$-20 $g/m^2$, and optionally 0.1 $g/m^2$-5 $g/m^2$. Moreover, the loading amount of the acid scavenging additive on the separator may also be 0.05 $g/m^2$-20 $g/m^2$, 0.3 $g/m^2$-19 $g/m^2$, 0.5 $g/m^2$-18 $g/m^2$, 0.7 $g/m^2$-17 $g/m^2$, 0.8 $g/m^2$-16 $g/m^2$, 0.9 $g/m^2$-15 $g/m^2$, 1.0 $g/m^2$-14 $g/m^2$, 1.5 $g/m^2$-12 $g/m^2$, 1.8 $g/m^2$-11 $g/m^2$, 2.0 $g/m^2$-10 $g/m^2$, 2.5 $g/m^2$-8 $g/m^2$, 2.9 $g/m^2$-7 $g/m^2$, 3.0 $g/m^2$-6 $g/m^2$, 3.5 $g/m^2$-5.5 $g/m^2$, 3.5 $g/m^2$-5.0 $g/m^2$, 3.6 $g/m^2$-4.8 $g/m^2$, 3.8 $g/m^2$-4.6 $g/m^2$, 3.9 $g/m^2$-4.3 $g/m^2$ and 4.0 $g/m^2$-4.7 $g/m^2$。

In the present application, the amount of the acid scavenging additive loaded on the separator can be adjusted according to the actual situation (e.g., according to the battery system and the acid scavenging ability of the selected acid scavenging additive). The loading amount of the acid scavenging additive on the separator can be set to be greater than or equal to 0.01 $g/m^2$, so that the content of the acidic substances in the battery system can be effectively reduced. On the other hand, the loading amount of the acid scavenging additive on the separator can be set to be less than or equal to 20 $g/m^2$, so that the acid scavenging effect can be fully guaranteed over a long period of time without significantly deteriorating the gas permeability of the separator and not significantly increasing the internal resistance of the battery.

In some embodiments, the thickness of the inorganic and/or organic particle layer can be 0.1 μm-10 μm, and optionally 0.5 μm-5 μm. Furthermore, the thickness of the inorganic and/or organic particle layer may be 0.2 μm-9 μm, 0.3 μm-8.5 μm, 0.4 μm-8 μm, 0.5 μm-7.5 μm, 0.6 μm-7.0 μm, 0.7 μm-6.5 μm, 0.8 μm-6.0 μm, 0.9 μm-5.5 μm, 1.0 μm-5.0 μm, 1.5 μm-4.5 μm, 2.0 μm-4.0 μm, 2.5 μm-3.8 μm, 2.7 μm-3.4 μm, and 2.8 μm-3.9 μm.

In the present application, the thickness of the inorganic and/or organic particle layer can be adjusted according to the actual situation (e.g., according to the battery system, the acid scavenging ability of the selected acid scavenging additive, the size of the acid scavenging additive, etc.). The thickness of the inorganic and/or organic particle layer can be set to be greater than or equal to 0.5 μm, so that the contact between the acid scavenging additive and the interface of the positive and negative electrodes can be effectively isolated. On the other hand, the thickness of the inorganic and/or organic particle layer can be set to be less than or equal to 10 μm, so that the acid scavenging effect can be guaranteed and the occurrence of side reactions in the battery can be reduced without significantly deteriorating the air permeability of the separator (not seriously blocking the pores of the separator) and significantly increasing the internal resistance of the battery.

In some embodiments, there is no particular limitation on the inorganic particle, as long as it has electronic insulating properties and appropriate mechanical strength. In this way, it can be ensured that the active materials of the positive and negative electrodes do not contact the acid scavenging additive on the separator, nor conduct electrons to the acid scavenging additive, so that the acid scavenging additive cannot undergo a redox reaction, and the stability of the acid scavenging additive on the separator is guaranteed. In the present application, from the viewpoint of improving the cycling performance and storage performance, especially the high temperature cycling performance and high temperature storage performance of the battery, the inorganic particle can include at least one selected from the group consisting of silica, alumina, boehmite, magnesia, titanium dioxide, zinc oxide, and magnesium aluminate oxide. Similarly, in the present application, there is no particular limitation on the organic particle, as long as it has electronic insulating properties and appropriate mechanical strength. From the viewpoint of improving the cycling performance and storage performance, especially the high temperature cycling performance and high temperature storage performance of the battery, the organic particle includes at least one selected from the group consisting of polyethylene oxide particles, polyvinylidene chloride particles, polyacrylonitrile particles, polyvinylidene fluoride particles, polymethyl methacrylate particles, and poly(vinylidene fluoride-hexafluoropropylene) copolymer particles.

In some embodiments, an electrolyte solution commonly used in the art can be used as the electrolyte solution. The electrolyte solution can include a solvent and an electrolyte salt. Optionally, the solvent may include a solvent commonly used in the art, for example, a solvent selected from at least one of a carbonate solvent, a sulfone solvent, an ether solvent, a sulfonate solvent, a sulfate solvent and a sulfite solvent. The electrolyte salt may include a lithium or sodium salt.

In some embodiments, a substrate commonly used in the art as a substrate of the separator can be used as the substrate. The substrate can include at least one selected from polyethylene, polypropylene, cellulose and polyimide. In the present application, the thickness of the substrate may be 1-20 μm. Optionally, the thickness of the substrate may be 2-15 μm, 2-10 μm, 2.5-9 μm, 3-8 μm, 3.5-8 μm, 4-7.5 μm, 4.5-6 μm, and 5-6.5 μm. By adopting the aforementioned thickness, the air permeability of the separator can be ensured, the energy density of the battery can be improved, and the like while the insulating properties are ensured.

A second aspect of the present application provides a battery module including the secondary battery provided by the first aspect of the present application.

A third aspect of the present application provides a battery pack including the battery module provided by the second aspect of the present application.

A fourth aspect of the present application provides an electrical apparatus including at least one selected from the secondary battery according to the first aspect of the present application, the battery module according to the second aspect of the present application, or the battery pack according to the third aspect of the present application.

The aforementioned battery module, battery pack, and electrical apparatus include the secondary battery of the present application, so that the cycling performance and storage performance, especially the high temperature cycling performance and high temperature storage performance of the battery can be significantly improved.

## DETAILED DESCRIPTION

The secondary battery of the present application can include a positive electrode sheet, a negative electrode sheet, a separator disposed between the positive electrode sheet and the negative electrode sheet, and an electrolyte solution. The secondary battery, battery module, battery pack, and electrical apparatus of the present application will be described in detail hereafter with reference to the drawings.

[negative electrode sheet]

The negative electrode sheet can include a negative current collector, and a negative electrode film sheet disposed on at least one surface of the negative current collector. The negative electrode film sheet can include a negative electrode active material, an optional binder, a conductive agent, and other adjuvants.

As an example, the negative current collector has two opposite surfaces in its own thickness direction, and the negative electrode film sheet is disposed on either or both of the opposite surfaces of the negative current collector.

In some embodiments, the negative current collector can be a metal foil or a composite current collector. For example, a copper foil can be used as the metal foil. The composite current collector may include a high molecular material substrate and a metal layer formed on at least one surface of the high molecular material substrate. The composite current collector can be formed by forming the metal material (e.g. copper, copper alloy, nickel, nickel alloy, titanium, titanium alloy, silver and silver alloy, etc.) on the high molecular material substrate (e.g. polypropylene (PP), polyethylene terephthalate (PET), polybutylene terephthalate (PBT), polystyrene (PS), polyethylene (PE), and the like substrates).

In some embodiments, a negative electrode active material for the battery well known in the art can be used as the negative electrode active material. As an example, the negative electrode active material may include at least one of the following materials: artificial graphite, natural graphite, soft carbon, hard carbon, silicon-based materials, tin-based materials, lithium titanate, and the like. The silicon-based material can be at least one selected from elemental silicon, a silicon oxide compound, a silicon-carbon composite, a silicon-nitrogen composite, and a silicon alloy. The tin-based material may be at least one selected from elemental tin, a tin oxide compound, and a tin alloy. However, the present application is not limited to these materials, and other conventional materials useful as negative electrode active materials for batteries can also be used. These negative electrode active materials may be used alone or in combination of two or more thereof.

Moreover, when the secondary battery is a sodium-ion battery, the negative electrode active material may include at least one selected from natural graphite, modified graphite, artificial graphite, graphene, carbon nanotubes, carbon nanofibers, porous carbon, tin, antimony, germanium, lead, ferric oxide, vanadium pentoxide, tin dioxide, titanium dioxide, molybdenum trioxide, elemental phosphorus, sodium titanate and sodium terephthalate.

In some embodiments, the negative electrode film sheet may further optionally include a binder. The binder may be at least one selected from styrene butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), and carboxymethyl chitosan (CMCS).

In some embodiments, the negative electrode film sheet may further optionally include a conductive agent. The conductive agent may be at least one selected from superconducting carbon, acetylene black, carbon black, Ketjen black, carbon nanotube, carbon nanorod, graphene, and carbon nanofiber.

In some embodiments, the negative electrode film sheet may further optionally include other adjuvants, for example, a thickener (e.g., sodium carboxymethylcellulose (CMC-Na)) and the like.

In some embodiments, the negative electrode sheet can be prepared by: dispersing the aforementioned components for preparing the negative electrode sheet, e.g., the negative electrode active material, the conductive agent, the binder and any other components in a solvent (e.g. deionized water) to form a negative electrode slurry; and coating the negative electrode slurry on a negative current collector, followed by oven drying, cold pressing and the like procedures, to obtain the negative electrode sheet. Alternatively, in another embodiment, the negative electrode sheet can be prepared by: casting the negative electrode slurry for forming the negative electrode film sheet on a separate carrier, and then laminating a film peeled from the carrier on the negative current collector.

[positive electrode sheet]

The positive electrode sheet includes a positive current collector and a positive electrode film sheet that is disposed on at least one surface of the positive current collector and includes a positive electrode active material. As an example, the positive current collector has two opposite surfaces in its own thickness direction, and the positive electrode film sheet is disposed on either or both of the opposite surfaces of the positive current collector.

The positive current collector can be made of a material having good electrical conductivity and mechanical strength. In some embodiments, an aluminum foil can be employed as the positive current collector.

The present application has no particular limitation on the specific type of the positive electrode active material, and a material that can be used as the material of the positive electrode of the secondary battery as known in the art can be used and can be selected by those skilled in the art according to actual requirements.

In some embodiments, the secondary battery may be a lithium-ion secondary battery. The positive electrode active material may be selected from lithium transition metal oxides and modified materials thereof. The aforementioned modified materials may be modified by doping and/or coating the lithium transition metal oxides. For example, the lithium transition metal oxide may be one or more selected from a lithium-cobalt oxide, a lithium-nickel oxide, a lithium-manganese oxide, a lithium-nickel-manganese oxide, a lithium-nickel-cobalt-manganese oxide, a lithium-nickel-cobalt-aluminum oxide, and a lithium-containing phosphate of an olivine structure.

As an example, the positive electrode active material of the secondary battery may be one or more selected from $LiCoO_2$, $LiNiO_2$, $LiMnO_2$, $LiMn_2O_4$, $LiNi_{1/3}Co_{1/3}Mn_{1/3}O_2$(NCM333), $LiNi_{0.5}Co_{0.2}Mn_{0.3}O_2$(NCM523), $LiNi_{0.6}Co_{0.2}Mn_{0.2}O_2$(NCM622), $LiNi_{0.8}Co_{0.1}Mn_{0.1}O_2$(NCM811), $LiNi_{0.85}Co_{0.15}Al_{0.05}O_2$, $LiFePO_4$(LFP) and $LiMnPO_4$.

Moreover, when the secondary battery is a sodium-ion battery, the positive electrode may include at least one positive electrode active material selected from the following: a layered transition metal oxide, a polyanionic compound, a Prussian blue-based compound, a sulfide, a nitride, a carbide, and a titanate. Optionally, the positive electrode active material includes, but is not limited to at least one selected from the group consisting of $NaCrO_2$, $Na_2Fe_2(SO_4)_3$, molybdenum disulfide, tungsten disulfide, vanadium disulfide, titanium disulfide, hexagonal boron nitride, carbon-doped hexagonal boron nitride, titanium carbide, tantalum carbide, molybdenum carbide, silicon carbide, $Na_2Ti_3O_7$, $Na_2Ti_6O_{13}$, $Na_4Ti_5O_{12}$, $Li_4Ti_5O_{12}$, and $NaTi_2(PO_4)_3$.

In some embodiments, the positive electrode film sheet may further optionally include a binder. There is no particular limitation on the type of the binder, and it can be selected by those skilled in the art according to actual requirements. As an example, the binder for the positive electrode film sheet may include one or more of polyvinylidene fluoride (PVDF) and polytetrafluoroethylene (PTFE).

In some embodiments, the positive electrode film sheet may further optionally include a conductive agent. There is no particular limitation on the type of the conductive agent, and it can be selected by those skilled in the art according to actual requirements. As an example, the conductive agent for the positive electrode film sheet can include one or more of graphite, superconducting carbon, acetylene black, carbon black, Ketjen black, carbon dots, carbon nanotubes, graphene and carbon nanofibers.

[electrolyte solution]

The electrolyte solution serves to conduct ions between the positive electrode and the negative electrode. The present application has no specific limitation on the type of the electrolyte solution, and it can be selected according to requirements. For example, the electrolyte solution may be in a liquid or gel state.

Moreover, the electrolyte solution according to the embodiment of the present application can include an additive. The additive may include an additive commonly used in the art. The additive may include, for example, halogenated alkylene carbonates (e.g., ethylene difluorocarbonate), pyridine, triethyl phosphite, triethanolamine, cyclic ethers, ethylenediamine, (poly)ethylene glycol dimethyl ethers, hexamethylphosphoric triamide, nitrobenzene derivatives, sulfur, quinoneimine dyes, N-substituted oxazolidinones, N,N-substituted imidazolidines, ethylene glycol dialkyl ethers, ammonium salts, pyrrole, 2-methoxyethanol or aluminum trichloride. At this time, based on the total weight of the electrolyte solution, the additive may be included in an amount of 0.1 mass% to 5 mass%, or the amount of the additive may be adjusted by those skilled in the art according to actual requirements.

Generally, the electrolyte solution can include an electrolyte salt and a solvent.

In an embodiment where the secondary battery is a lithium-ion battery, the electrolyte salt may include at least one selected from $LiPF_6$, $LiBF_4$, $LiN(SO_2F)_2$ (LiFSI), $LiN(CF_3SO_2)_2$ (LiTFSI), $LiClO_4$, $LiAsF_6$, $LiB(C_2O_4)_2$ (LiBOB), and $LiBF_2C_2O_4$ (LiDFOB).

In an embodiment where the secondary battery is a sodium-ion battery, the electrolyte salt may include at least one selected from $NaPF_6$, $NaClO_4$, $NaBCl_4$, $NaSO_3CF_3$ and $Na(CH_3)C_6H_4SO_3$.

In some embodiments, in addition to the aforementioned solvents such as the low viscosity solvents and the high dielectric constant solvents, the electrolyte solution may further include other solvents commonly used in the art as desired, such as at least one selected from 1,4-butyrolactone, sulfolane, dimethyl sulfone, methyl ethyl sulfone, diethyl sulfone, and the like.

[separator]

The separator described above is employed as the separator of the present application. Furthermore, the separator of the present application can be used in combination with other separators commonly used in the art, as required.

[outer package]

In some embodiments, the secondary battery may include an outer package for encapsulating the positive electrode sheet, the negative electrode sheet and the electrolyte solution. As an example, the positive electrode sheet, the negative electrode sheet and the separator can be laminated or rolled to form a battery of a laminated structure or a rolled structure, and the battery is packaged in the outer package; and the electrolyte solution is infiltrated into the battery. The number of batteries in the secondary battery can be one or several, which can be adjusted as desired.

In some embodiments, the outer package of the secondary battery may be a soft package, such as a bag-type soft package. The material of the soft package can be plastic, and for example may include one or more of polypropylene PP, polybutylene terephthalate PBT, polybutylene succinate PBS, etc. The outer package of the secondary battery can also be a hard shell, such as an aluminum shell.

In some embodiments, the positive electrode sheet, the negative electrode sheet and the separator can be made into an electrode assembly by a winding process or a lamination process.

The present application has no particular limitation on the shape of the secondary battery, which can be cylindrical, square or any other shape. For example, Fig. 1 shows a secondary battery 5 with a square structure as an example.

[battery module]

In the second aspect of the present application, secondary batteries may be assembled into a battery module, the number of secondary batteries contained in the battery module can be multiple, and the specific number can be adjusted according to the application and capacity of the battery module.

Fig. 2 shows a battery module 4 as an example. Referring to Fig. 2, in the battery module 4, multiple secondary batteries 5 may be sequentially arranged along a length direction of the battery module 4. Of course, they can also be arranged in any other way. The multiple secondary batteries 5 can be further fixed by fasteners.

Optionally, the battery module 4 may further include a case having an accommodating space in which the multiple secondary batteries 5 are accommodated.

[battery pack]

In the third aspect of the present application, the battery modules provided by the second aspect of the present application can also be assembled into a battery pack, and the number of battery modules included in the battery pack can be adjusted according to the application and capacity of the battery pack.

Figs. 3 and 4 show a battery pack 1 as an example. Referring to Figs. 3 and 4, the battery pack 1 may include a battery box and multiple battery modules 4 disposed in the battery box. The battery box includes an upper box body 2 and a lower box body 3, the upper box body 2 can cover the lower box body 3 to form a closed space for accommodating the battery modules 4. The multiple battery modules 4 may be arranged in the battery box in any manner.

[electrical apparatus]

The fourth aspect of the present application further provides an electrical apparatus, which includes the secondary battery of the first aspect of the present application, and the secondary battery provides power for the electrical apparatus. The electrical apparatus may be, but is not limited to, a mobile device (e.g. a mobile phone, a laptop), an electric vehicle (e.g. an all-electric vehicle, a hybrid electric vehicle, a plug-in hybrid electric vehicle, an electric bicycle, an electric scooter, an electric golf cart, an electric truck, etc.), an electric train, a ship, a satellite, an energy storage system, etc.

For the electrical apparatus, the secondary battery, the battery module, or the battery pack can be selected according to the use requirements of the electrical apparatus.

Fig. 5 shows an electrical apparatus as an example. The electrical apparatus is an all-electric vehicle, a hybrid electric vehicle or a plug-in hybrid electric vehicle, and the like. In order to meet the requirements of the electrical apparatus for high power and high energy density of secondary batteries, a battery pack or a battery module may be used.

As another example, the electrical apparatus may be a mobile phone, a tablet, a laptop, etc. The electrical apparatus is generally required to be light and thin, and can use a secondary battery as a power source.

## Examples

Examples of the present application will be described hereinafter. The following examples are illustrative and only for explaining the present application, and should not be construed as limiting the present application. If no specific technology or condition is indicated in the examples, it shall be carried out according to the technology or condition described in the literature in the art or according to product specifications. All of the used agents or instruments which are not specified with the manufacturer are conventional commercially-available products.

The lithium/sodium-ion batteries of Examples 1-48 and Comparative Examples 1-5 are all prepared according to the following method, and the composition of the specific separator is shown in Table 1 below.

(1) Preparation of positive electrode sheet of lithium-ion battery

The positive electrode active material of lithium manganate, the conductive agent of acetylene black and the binder of polyvinylidene fluoride (PVDF) were dissolved in a solvent of N-methylpyrrolidone (NMP) according to the mass ratio of 94:3:3, and mixed under sufficient stirring to obtain a positive electrode slurry; thereafter, the positive electrode slurry was evenly coated on an aluminum foil as the positive current collector, and then subjected to oven drying, cold pressing and slitting to obtain a positive electrode sheet.

(2) Preparation of negative electrode sheet of lithium-ion battery

The negative electrode active material of artificial graphite, the conductive agent of acetylene black, the binder of styrene butadiene rubber (SBR) and the thickener of sodium carboxymethylcellulose (CMC-Na) were dissolved in a solvent of deionized water according to the mass ratio of 95:2:2: 1. They were uniformly mixed with the solvent of deionized water to prepare a negative electrode slurry; and thereafter, the negative electrode slurry was evenly coated on a copper foil as the negative current collector for one or more times, and then subjected to oven drying, cold pressing and slitting to obtain a negative electrode sheet.

(3) Preparation of electrolyte solution of lithium-ion battery

In a glove box in an argon atmosphere ($H_2O$ < 0.1 ppm, $O_2$ < 0.1 ppm), organic solvents of ethylene carbonate (EC)/ethyl methyl carbonate (EMC) were uniformly mixed according to a volume ratio of 3/7 to obtain a mixed solvent. Relative to the mass of the mixed solvent, 12.5% by mass of $LiPF_6$, and 1% by mass of 1,3-propanesultone (PS), 0.5% by mass of ethylene sulfate (DTD) and 0.5% by mass of vinylene carbonate (VC) as additives were added, and stirred uniformly to obtain a corresponding electrolyte solution.

(4) Preparation of positive electrode sheet of sodium-ion battery

The positive electrode active material of sodium vanadate phosphate, the conductive agent of acetylene black and the binder of polyvinylidene fluoride (PVDF) were dissolved in a solvent of N-methylpyrrolidone (NMP) according to the mass ratio of 80:15:3, and mixed under sufficient stirring to obtain a positive electrode slurry; thereafter, the positive electrode slurry was evenly coated on an aluminum foil as the positive current collector, and then subjected to oven drying, cold pressing and slitting to obtain a positive electrode sheet.

(5) Preparation of negative electrode sheet of sodium-ion battery

The active material of hard carbon, the conductive agent of acetylene black, the binder of styrene butadiene rubber and the thickener of sodium carboxymethylcellulose (CMC-Na) were dissolved in a solvent of deionized water according to the mass ratio of 91.6: 1.6:4.8:2. They were uniformly mixed with the solvent of deionized water to prepare a negative electrode slurry; and thereafter, the negative electrode slurry was evenly coated on an aluminum foil as the negative current collector for one or more times, and then subjected to oven drying, cold pressing and slitting to obtain

a negative electrode sheet.

(6) Preparation of electrolyte solution of sodium-ion battery

In a glove box in an argon atmosphere ($H_2O$ < 0.1 ppm, $O_2$ < 0.1 ppm), organic solvents of ethylene carbonate (EC)/ethyl methyl carbonate (EMC) were uniformly mixed according to a volume ratio of 3/7. Relative to the mass of the mixed solvent, 13.8% by mass of a lithium salt of $NaPF_6$, and 1% by mass of VC as an additive were added, and stirred uniformly to obtain a corresponding electrolyte solution.

(7) Preparation of separator

Layered coating: firstly the acid scavenging additive was dissolved or dispersed in deionized water, and then the dissolved or dispersed acid scavenging additive was coated on the substrate of polyethylene by gravure method to obtain the acid scavenging additive layer, and a desired loading amount can be obtained by coating for many times. Then, a non-conductive raw material of inorganic and/or organic particles and a binder of water-based polyacrylate were dispersed in deionized water according to a mass ratio of 99:1 to obtain inorganic and/or organic slurry, and then the inorganic and/or organic slurry was coated over the acid scavenging additive layer. A desired thickness of the inorganic and/or organic particle layer could be obtained by adjusting the coating mass of the inorganic and/or organic slurry.

Mixed coating:

The mixed coating of separators of Examples 30-33, 39, 40, 42, 45 and 47 (the mass ratio of polyvinylidene fluoride particles to boehmite in Example 47 was 1:1): the acid scavenging additive, the non-conductive raw material of inorganic and/or organic particles and the binder of water-based polyacrylate were dispersed in deionized water according to a mass ratio of 10:89:1 to obtain an inorganic and/or organic slurry, then the inorganic and/or organic slurry was coated on the substrate of polyethylene, and a desired thickness of an inorganic and/or organic particle layer could be obtained by adjusting the coating mass of the inorganic and/or organic slurry.

The mixed coating of the separator of Example 35: the acid scavenging additive, non-conductive inorganic powder and the binder of water-based polyacrylate were dispersed in deionized water according to a mass ratio of 30:69:1 to obtain an inorganic slurry, then the inorganic slurry was coated on the substrate of polyethylene, and a desired thickness of an inorganic particle layer could be obtained by adjusting the coating mass of the inorganic slurry.

The mixed coating of the separator of Example 36: the acid scavenging additive, non-conductive inorganic powder and the binder of water-based polyacrylate were dispersed in deionized water according to a mass ratio of 40:59:1 to obtain an inorganic slurry, then the inorganic slurry was coated on the substrate of polyethylene, and a desired thickness of an inorganic particle layer could be obtained by adjusting the coating mass of the inorganic slurry.

In the examples of the present application, the maximum dissolution amount of each acid scavenging additive in the electrolyte solution at 25°C was obtained by measuring the maximum dissolution amount of each acid scavenging additive in a corresponding electrolyte solution at 25°C and dividing the mass by the total mass of the dissolved acid scavenging additive and the electrolyte solution.

(8) Preparation of lithium/sodium-ion battery

The positive electrode sheet, the separator and the negative electrode sheet were stacked sequentially, so that the separator was positioned between the positive electrode sheet and the negative electrode sheet to separate them, and then they were wound to obtain an electrode assembly; the electrode assembly was put into a battery case and dried, and then injected with the aforementioned electrolyte solution, and thereafter subjected to processes such as chemical formation and standing to obtain the lithium/sodium-ion battery.

Table 1

| Serial Number | Battery system | Substrate | Acid scavenging additive (maximum dissolution amount in the aforementioned electrolyte solution at 25°C) | Loading amount of the acid scavenging additive ($g/m^2$) | Coating manner of the acid scavenging additive | Inorganicand/ororganic particles | Thickness of inorganic and/or organic particle layer (μm) |
|---|---|---|---|---|---|---|---|
| Comparative Example 1 | Lithium-ion battery | 7μm PE | / | / | Layered coating | boehmite | 2 |
| Comparative Example 2 | Sodium-ion battery | 7μm PE | / | / | Layered coating | boehmite | 3 |
| Comparative Example 3 | Lithium-ion battery | 7μm PE | Pyridine (> 10% by mass) | 0.5 | Layered coating | boehmite | 2 |
| Comparative Example 4 | Lithium-ion battery | 7μm PE | Triphenylphosphine (> 2% by mass) | 0.5 | Layered coating | boehmite | 2 |
| Comparative Example 5 | Lithium-ion battery | 7μm PE | Lithium 2,2'-bipyridine-4,4'-dicarboxylate (< 0.2% by mass) | 0.5 | Layered coating | / | / |
| Example 1 | Lithium-ion battery | 7μm PE | Lithium 2,2'-bipyridine-4,4'-dicarboxylate (< 0.2% by mass) | 0.01 | Layered coating | boehmite | 2 |
| Example 2 | Lithium-ion battery | 7μm PE | Lithium 2,2'-bipyridine-4,4'-dicarboxylate (< 0.2% by mass) | 0.05 | Layered coating | boehmite | 2 |
| Example 3 | Lithium-ion battery | 7μm PE | Lithium 2,2'-bipyridine-4,4'-dicarboxylate (< 0.2% by mass) | 0.1 | Layered coating | boehmite | 2 |
| Example 4 | Lithium-ion battery | 7μm PE | Lithium 2,2'-bipyridine-4,4'-dicarboxylate (< 0.2% by mass) | 0.5 | Layered coating | boehmite | 2 |

(continued)

| Serial Number | Battery system | Substrate | Acid scavenging additive (maximum dissolution amount in the aforementioned electrolyte solution at 25°C) | Loading amount of the acid scavenging additive ($g/m^2$) | Coating manner of the acid scavenging additive | Inorganic and/or organic particles | Thickness of inorganic and/or organic particle layer (μm) |
|---|---|---|---|---|---|---|---|
| Example 5 | Lithium-ion battery | 7μm PE | Lithium 2,2'-bipyridine-4,4'-dicarboxylate (< 0.2% by mass) | 1 | Layered coating | boehmite | 2 |
| Example 6 | Lithium-ion battery | 7μm PE | Lithium 2,2'-bipyridine-4,4'-dicarboxylate (< 0.2% by mass) | 3 | Layered coating | boehmite | 2 |
| Example 7 | Lithium-ion battery | 7μm PE | Lithium 2,2'-bipyridine-4,4'-dicarboxylate (< 0.2% by mass) | 5 | Layered coating | boehmite | 2 |
| Example 8 | Lithium-ion battery | 7μm PE | Lithium 2,2'-bipyridine-4,4'-dicarboxylate (< 0.2% by mass) | 8 | Layered coating | boehmite | 2 |
| Example 9 | Lithium-ion battery | 7μm PE | Lithium 2,2'-bipyridine-4,4'-dicarboxylate (< 0.2% by mass) | 10 | Layered coating | boehmite | 2 |
| Example 10 | Lithium-ion battery | 7μm PE | Sodium 2,2'-bipyridine-4,4'-dicarboxylate (< 0.2% by mass) | 0.5 | Layered coating | boehmite | 2 |
| Example 11 | Lithium-ion battery | 7μm PE | Potassium 2,2'-bipyridine-4,4'-dicarboxylate (< 0.2% by mass) | 0.5 | Layered coating | boehmite | 2 |
| Example 12 | Lithium-ion battery | 7μm PE | Lithium 2-hydroxypyridine-4-carboxylate (< 0.2% by mass) | 0.5 | Layered coating | boehmite | 2 |
| Example 13 | Lithium-ion battery | 7μm PE | Diethylphenylphosphine (< 0.5% by mass) | 0.5 | Layered coating | boehmite | 2 |

(continued)

| Serial Number | Battery system | Substrate | Acid scavenging additive (maximum dissolution amount in the aforementioned electrolyte solution at 25°C) | Loading amount of the acid scavenging additive ($g/m^2$) | Coating manner of the acid scavenging additive | Inorganic and/or organic particles | Thickness of inorganic and/or organic particle layer (μm) |
|---|---|---|---|---|---|---|---|
| Example 14 | Lithium-ion battery | 7μm PE | Trihexylphosphine (< 0.5% by mass) | 0.5 | Layered coating | boehmite | 2 |
| Example 15 | Lithium-ion battery | 7μm PE | Tri-n-octylphosphine (< 0.5% by mass) | 0.5 | Layered coating | boehmite | 2 |
| Example 16 | Lithium-ion battery | 12 μm PP | Lithium isonicotinate (< 0.2% by mass) | 0.5 | Layered coating | boehmite | 2 |
| Example 17 | Lithium-ion battery | 12 μm cellulose | Sodium isonicotinate (< 0.2% by mass) | 0.5 | Layered coating | boehmite | 2 |
| Example 18 | Lithium-ion battery | 12 μm PI | Potassium isonicotinate (< 0.2% by mass) | 0.5 | Layered coating | boehmite | 2 |
| Example 19 | Lithium-ion battery | 7μm PE | Potassium 2,2'-bipyridine-4,4'-dicarboxylate (< 0.2% by mass) | 0.5 | Layered coating | boehmite | 0.1 |
| Example 20 | Lithium-ion battery | 7μm PE | Potassium 2,2'-bipyridine-4,4'-dicarboxylate (< 0.2% by mass) | 0.5 | Layered coating | boehmite | 0.3 |
| Example 21 | Lithium-ion battery | 7μm PE | Potassium 2,2'-bipyridine-4,4'-dicarboxylate (< 0.2% by mass) | 0.5 | Layered coating | boehmite | 0.5 |
| Example 22 | Lithium-ion battery | 7μm PE | Potassium 2,2'-bipyridine-4,4'-dicarboxylate (< 0.2% by mass) | 0.5 | Layered coating | boehmite | 1 |

(continued)

| Serial Number | Battery system | Substrate | Acid scavenging additive (maximum dissolution amount in the aforementioned electrolyte solution at 25°C) | Loading amount of the acid scavenging additive ($g/m^2$) | Coating manner of the acid scavenging additive | Inorganic and/or organic particles | Thickness of inorganic and/or organic particle layer (μm) |
|---|---|---|---|---|---|---|---|
| Example 23 | Lithium-ion battery | 7μm PE | Potassium 2,2'-bipyridine-4,4'-dicarboxylate (< 0.2% by mass) | 0.5 | Layered coating | boehmite | 5 |
| Example 24 | Lithium-ion battery | 7μm PE | Potassium 2,2'-bipyridine-4,4'-dicarboxylate (< 0.2% by mass) | 0.5 | Layered coating | boehmite | 8 |
| Example 25 | Lithium-ion battery | 7μm PE | Potassium 2,2'-bipyridine-4,4'-dicarboxylate (< 0.2% by mass) | 0.5 | Layered coating | boehmite | 10 |
| Example 26 | Lithium-ion battery | 7μm PE | Potassium 2,2'-bipyridine-4,4'-dicarboxylate (< 0.2% by mass) | 0.5 | Layered coating | alumina | 2 |
| Example 27 | Lithium-ion battery | 7μm PE | Potassium 2,2'-bipyridine-4,4'-dicarboxylate (< 0.2% by mass) | 0.5 | Layered coating | Titanium dioxide | 2 |
| Example 28 | Lithium-ion battery | 7μm PE | Lithium 2,2'-bipyridine-4,4'-dicarboxylate (< 0.2% by mass) | 19 | Layered coating | boehmite | 2 |
| Example 29 | Lithium-ion battery | 7μm PE | Lithium 2,2'-bipyridine-4,4'-dicarboxylate (< 0.2% by mass) | 23 | Layered coating | boehmite | 2 |
| Example 30 | Lithium-ion battery | 7μm PE | Sodium 2,2'-bipyridine-4,4'-dicarboxylate (< 0.2% by mass) | 0.5 | Mixed coating | boehmite | 2 |
| Example 31 | Lithium-ion battery | 7μm PE | Lithium isonicotinate (< 0.2% by mass) | 0.5 | Mixed coating | boehmite | 2 |

(continued)

| Serial Number | Battery system | Substrate | Acid scavenging additive (maximum dissolution amount in the aforementioned electrolyte solution at 25°C) | Loading amount of the acid scavenging additive ($g/m^2$) | Coating manner of the acid scavenging additive | Inorganic and/or organic particles | Thickness of inorganic and/or organic particle layer (μm) |
|---|---|---|---|---|---|---|---|
| Example 32 | Lithium-ion battery | 7μm PE | Trihexylphosphine (< 0.5% by mass) | 0.5 | Mixed coating | boehmite | 2 |
| Example 33 | Lithium-ion battery | 7μm PE | Tri-n-octylphosphine (< 0.5% by mass) | 0.5 | Mixed coating | boehmite | 2 |
| Example 34 | Lithium-ion battery | 7μm PE | Sodium 2,2'-bipyridine-4,4'-dicarboxylate (< 0.2% by mass) | 0.5 | Layered coating | boehmite | 12 |
| Example 35 | Lithium-ion battery | 7μm PE | Sodium 2,2'-bipyridine-4,4'-dicarboxylate (< 0.2% by mass) | 0.5 | Mixed coating | boehmite | 8 |
| Example 36 | Lithium-ion battery | 7μm PE | Sodium 2,2'-bipyridine-4,4'-dicarboxylate (< 0.2% by mass) | 0.5 | Mixed coating | boehmite | 12 |
| Example 37 | Lithium-ion battery | 7μm PE | Sodium 2,2'-bipyridine-4,4'-dicarboxylate (< 0.2% by mass) | 0.5 | Layered coating | Titanium dioxide | 2 |
| Example 38 | Lithium-ion battery | 7μm PE | Sodium 2,2'-bipyridine-4,4'-dicarboxylate (< 0.2% by mass) | 0.5 | Layered coating | magnesium aluminate oxide | 2 |
| Example 39 | Lithium-ion battery | 7μm PE | Sodium 2,2'-bipyridine-4,4'-dicarboxylate (< 0.2% by mass) | 0.5 | Mixed coating | Titanium dioxide | 2 |
| Example 40 | Lithium-ion battery | 7μm PE | Sodium 2,2'-bipyridine-4,4'-dicarboxylate (< 0.2% by mass) | 0.5 | Mixed coating | magnesium aluminate oxide | 2 |

(continued)

| Serial Number | Battery system | Substrate | Acid scavenging additive (maximum dissolution amount in the aforementioned electrolyte solution at 25°C) | Loading amount of the acid scavenging additive ($g/m^2$) | Coating manner of the acid scavenging additive | Inorganicand/ororganic particles | Thickness of inorganic and/or organic particle layer (μm) |
|---|---|---|---|---|---|---|---|
| Example 41 | Sodium-ion battery | 7μm PE | Lithium 2,2'-bipyridine-4,4'-dicarboxylate (< 0.2% by mass) | 0.5 | Layered coating | boehmite | 2 |
| Example 42 | Sodium-ion battery | 7μm PE | Sodium 2,2'-bipyridine-4,4'-dicarboxylate (< 0.2% by mass) | 0.5 | Mixed coating | boehmite | 2 |
| Example 43 | Lithium-ion battery | 7μm PE | Poly(4-vinylpyridine) (weight average molecular weight = 80,000) (< 0.1% by mass) | 0.5 | Layered coating | boehmite | 2 |
| Example 44 | Sodium-ion battery | 7μm PE | Lithium 2,2'-bipyridine-4,4'-dicarboxylate (weight average molecular weight = 80,000) (< 0.1% by mass) | 0.5 | Layered coating | boehmite | 2 |
| Example 45 | Lithium-ion battery | 7μm PE | Lithium 2,2'-bipyridine-4,4'-dicarboxylate (< 0.2% by mass) | 0.5 | Mixed coating | polyvinylidene fluoride particles | 2 |
| Example 46 | Lithium-ion battery | 7μm PE | Lithium 2,2'-bipyridine-4,4'-dicarboxylate (< 0.2% by mass) | 0.5 | Layered coating | polyvinylidene fluoride particles | 2 |
| Example 47 | Lithium-ion battery | 7μm PE | Lithium 2,2'-bipyridine-4,4'-dicarboxylate (< 0.2% by mass) | 0.5 | Mixed coating | polyvinylidene fluoride particles and boehmite (in a mass ratio of 1:1) | 2 |
| Example 48 | Lithium-ion battery | 7μm PE | Lithium 2,2'-bipyridine-4,4'-dicarboxylate (< 0.2% by mass) | 0.5 | Layered coating | polyvinylidene fluoride particles and boehmite (in a mass ratio of 1:1) | 2 |

A testing process of relevant parameters was described hereafter.

1. Air permeability of separator

Air permeability referred to the time it takes for a certain volume of air to pass through a unit area of paper and cardboard under unit pressure under specified conditions. The air permeability test of the separator was conducted with reference to GB/T458-2008, the air column volume was 100 cc, and the test area was 1 square inch.

2. Internal resistance of battery (mQ)

Lithium-ion battery: at 25°C, a lithium-ion battery was charged to 4.3 V at a constant current of 1 C, then charged at a constant voltage of 4.3 V until the current was less than 0.05 C, and then discharged at 1 C for 30 min, that is, the electric quantity of the battery was adjusted to 50% SOC. Afterwards, the positive and negative probes of a TH2523A alternating current internal resistance tester were made in contact with positive and negative electrodes of the battery respectively, so as to read an internal resistance value of the battery through the internal resistance tester.

Sodium-ion battery: at 25°C, a sodium-ion battery was charged to 4.2 V at a constant current of 1 C, then charged at a constant voltage of 4.2 V until the current was less than 0.05 C, and then discharged at 1 C for 30 min, that is, the electric quantity of the battery was adjusted to 50% SOC. Afterwards, the positive and negative probes of a TH2523A alternating current internal resistance tester were made in contact with positive and negative electrodes of the battery respectively, so as to read an internal resistance value of the battery through the internal resistance tester.

3. Capacity retention rate after 800 cycles at 45°C

Lithium-ion battery: at 45°C, a lithium-ion battery was charged to 4.3 V at a constant current of 1 C, then charged at a constant voltage of 4.3 V until the current was less than 0.05 C, and then discharged to 3.0 V at a constant current of 1 C, which was one cycle of charging and discharging process. Charging and discharging were repeated in this way, and a capacity retention rate of the lithium-ion battery after 800 cycles was calculated.

The capacity retention rate (%) of the lithium-ion battery after 800 cycles at 45°C = (discharge capacity of the 800th cycle/discharge capacity of the first cycle) × 100%.

Sodium-ion battery: at 45°C, a sodium-ion battery was charged to 4.2 V at a constant current of 1 C, then charged at a constant voltage of 4.2 V until the current was less than 0.05 C, and then discharged to 2.0 V at a constant current of 1 C, which was one cycle of charging and discharging process. Charging and discharging were repeated in this way, and a capacity retention rate of the sodium-ion battery after 800 cycles was calculated.

The capacity retention rate (%) of the sodium-ion battery after 800 cycles at 45°C = (discharge capacity of the 800th cycle/discharge capacity of the first cycle) x 100%.

4. Volume expansion rate after storage at 60°C for 50 days

Lithium-ion battery: at 25°C, a lithium-ion battery was charged to 4.3 V at a constant current of 1 C, and then charged at a constant voltage of 4.3 V until the current was 0.05 C, and at this time the volume of the lithium-ion battery was tested and recorded as V21; then, the fully-charged lithium-ion battery was placed into a 60°C thermostat and stored for 50 days, and then the volume of the lithium-ion battery was tested by a drainage method and recorded as V31.

Volume expansion rate (%) of the lithium-ion battery after storage at 60°C for 50 days = (V31 - V21)/V21 x 100%.

Sodium-ion battery: at 25°C, a sodium-ion battery was charged to 4.2 V at a constant current of 1 C, and then charged at a constant voltage of 4.2 V until the current was 0.05 C, and at this time the volume of the sodium-ion battery was tested and recorded as V22; then, the fully-charged sodium-ion battery was placed into a 60°C thermostat and

stored for 50 days, and then the volume of the sodium-ion battery was tested by a drainage method and recorded as V32.

$$\text{Volume expansion rate (\%) of the sodium-ion battery after storage at 60°C for 50 days} = (V32 - V22)/V22 \times 100\%.$$

Table 2 below showed the test data for Comparative Examples 1, 3 and 4 and Example 4.

Table 2

| Serial Number | Air permeability of separator (sec) | Internal resistance of battery (mΩ) | Capacity retention rate (%) after 800 cycles at 45°C | Volume expansion rate (%) after storage at 60°C for 50 days |
|---|---|---|---|---|
| Comparative Example 1 | 150 | 5.13 | 62 | 10 |
| Comparative Example 3 | 155 | 5.30 | 70 | 50 |
| Comparative Example 4 | 153 | 5.23 | 73 | 46 |
| Example 4 | 160 | 5.47 | 94 | 5 |

In Comparative Example 1, no acid scavenging additive was introduced, and the cycling performance of the battery was poor. This was mainly because the electrolyte solution reacted with the trace amount of water in the battery system at a high temperature to form acids. These acids reacted at the interface of the positive and negative electrodes, resulting in instability of the interface. Therefore, it was necessary to continuously consume the electrolyte solution to repair the interface of the positive and negative electrodes. Therefore, the high temperature cycling performance of the battery was poor. At the same time, the acids would also aggravate the dissolution of transition metals in the positive electrode active material, thereby further deteriorating the high temperature performance of the battery.

In Comparative Examples 3 and 4, pyridine and triphenylphosphine were added as the acid scavenging additive, so that the content of acidic substances in the electrolyte solution could be suppressed and the cycling performance of the battery was slightly improved. However, pyridine and triphenylphosphine were easily dissolved in the electrolyte solution, and then migrated to the interface of the positive and negative electrodes and underwent a redox reaction to generate gaseous products, resulting in the deterioration of the high temperature storage performance of the battery. At the same time, the acid scavenging additive was continuously consumed, resulting in no protection by the acid scavenging additive was existed in the later period of the cycle of the battery and thus limiting the effect of improving the cycling performance.

The lithium 2,2'-bipyridine-4,4'-dicarboxylate in Example 4 could significantly improve the cycling performance of the battery, which was mainly due to the following reasons. During a charging and discharging process, the acidic substances in the electrolyte solution reacted with the acid scavenging additive of lithium 2,2'-bipyridine-4,4'-dicarboxylate on the separator, so that the acid content in the battery system could be controlled at a relatively low level. Furthermore, in the present application, the acid scavenging additive of lithium 2,2'-bipyridine-4,4'-dicarboxylate was disposed in the form of a separate layer between the substrate and the inorganic and/or organic particle layer, and had low solubility in the electrolyte solution. Therefore, the acid scavenging additive of lithium 2,2'-bipyridine-4,4'-dicarboxylate could not be in direct contact with positive and negative electrodes, so that there was almost no electrochemical redox reaction between them. This ensured that the acid scavenging additive of lithium 2,2'-bipyridine-4,4'-dicarboxylate was present in the battery system throughout the life cycle of the battery. Therefore, the high temperature cycling performance and high temperature storage performance of the battery could be significantly improved.

Table 3 below showed the test data for Comparative Example 1 and Examples 1-18.

Table 3

| Serial Number | Air permeability of separator (sec) | Internal resistance of battery (mΩ) | Capacity retention rate (%) after 800 cycles at 45°C |
|---|---|---|---|
| Comparative Example 1 | 150 | 5.13 | 62 |

(continued)

| Serial Number | Air permeability of separator (sec) | Internal resistance of battery (mΩ) | Capacity retention rate (%) after 800 cycles at 45°C |
|---|---|---|---|
| Example 1 | 151 | 5.16 | 80 |
| Example 2 | 154 | 5.26 | 85 |
| Example 3 | 156 | 5.33 | 88 |
| Example 4 | 160 | 5.47 | 94 |
| Example 5 | 195 | 6.67 | 94 |
| Example 6 | 248 | 8.48 | 96 |
| Example 7 | 361 | 12.34 | 95 |
| Example 8 | 429 | 14.67 | 96 |
| Example 9 | 570 | 19.49 | 94 |
| Example 10 | 157 | 5.37 | 93 |
| Example 11 | 161 | 5.50 | 94 |
| Example 12 | 152 | 5.20 | 92 |
| Example 13 | 158 | 5.40 | 92 |
| Example 14 | 156 | 5.33 | 93 |
| Example 15 | 159 | 5.44 | 93 |
| Example 16 | 173 | 5.91 | 91 |
| Example 17 | 143 | 4.89 | 93 |
| Example 18 | 173 | 5.91 | 94 |

By comparing Comparative Example 1 and Examples 1-18, it could be seen that coating the acid scavenging additive of the present application on the separator could effectively improve the cycling performance of the battery at 45°C; however, coating too much would block the pores of the separator, resulting in the increase in the air permeability value, a deterioration in the air permeability of the separator, and a significant deterioration in the internal resistance of the battery. The loading amount of the acid scavenging additive on the separator was 0.01 $g/m^2$-20 $g/m^2$, and preferably 0.1 $g/m^2$-5 $g/m^2$.

Table 4 below showed the test data of Comparative Examples 1, 2 and 5, and Examples 11 and 19-47.

Table 4

| Serial Number | Air permeability of separator (sec) | Internal resistance of battery (mΩ) | Capacity retention rate (%) after 800 cycles at 45°C | Volume expansion rate (%) after storage at 60°C for 50 days |
|---|---|---|---|---|
| Comparative Example 1 | 150 | 5.13 | 62 | 10 |
| Comparative Example 2 | 150 | 3.28 | 55 | 30 |
| Comparative Example 5 | 145 | 4.96 | 75 | 38 |
| Example 11 | 161 | 5.50 | 94 | 5 |
| Example 19 | 146 | 4.99 | 78 | 20 |
| Example 20 | 149 | 5.09 | 85 | 15 |
| Example 21 | 155 | 5.30 | 88 | 10 |

(continued)

| Serial Number | Air permeability of separator (sec) | Internal resistance of battery (mΩ) | Capacity retention rate (%) after 800 cycles at 45°C | Volume expansion rate (%) after storage at 60°C for 50 days |
|---|---|---|---|---|
| Example 22 | 157 | 5.37 | 90 | 7 |
| Example 23 | 260 | 8.89 | 93 | 5 |
| Example 24 | 340 | 11.62 | 94 | 5 |
| Example 25 | 430 | 14.70 | 95 | 5 |
| Example 26 | 155 | 5.30 | 94 | 5 |
| Example 27 | 164 | 5.61 | 93 | 5 |
| Example 28 | 732 | 25.03 | 91 | 18 |
| Example 29 | 835 | 28.55 | 90 | 24 |
| Example 30 | 145 | 4.96 | 92 | 5 |
| Example 31 | 147 | 5.03 | 94 | 5 |
| Example 32 | 149 | 5.09 | 93 | 5 |
| Example 33 | 142 | 4.85 | 95 | 5 |
| Example 34 | 525 | 17.95 | 94 | 5 |
| Example 35 | 326 | 11.15 | 94 | 5 |
| Example 36 | 506 | 17.30 | 95 | 5 |
| Example 37 | 162 | 5.54 | 92 | 5 |
| Example 38 | 167 | 5.71 | 91 | 5 |
| Example 39 | 156 | 5.33 | 93 | 5 |
| Example 40 | 148 | 5.06 | 95 | 5 |
| Example 41 | 160 | 3.50 | 87 | 10 |
| Example 42 | 145 | 3.17 | 90 | 10 |
| Example 43 | 163 | 5.57 | 91 | 5 |
| Example 44 | 165 | 3.61 | 89 | 10 |
| Example 45 | 172 | 5.88 | 93 | 5 |
| Example 46 | 182 | 6.22 | 92 | 5 |
| Example 47 | 166 | 5.68 | 93 | 5 |
| Example 48 | 175 | 5.98 | 95 | 5 |

From the comparison of Comparative Examples 1 and 5 and Examples 11 and 19-27, it could be seen that potassium 2,2'-bipyridine-4,4'-dicarboxylate is not redox-resistant. If it was not protected by the inorganic and/or organic particle layer as the overlying layer, it would be in direct contact with the positive and negative electrodes, and underwent a redox reaction, resulting in gas production of the battery at a high temperature. At the same time, this would cause the acid scavenging additive to be continuously consumed, resulting in no protection by the acid scavenging additive in the later period of the cycle of the battery, so that the improvement of the cycling performance of the battery was not obvious. Therefore, it could be seen that the barrier effect of the inorganic and/or organic particle layer would affect the improvement effect of the acid scavenging additive on the performance of the battery.

It could be seen from Table 4 that the volume expansion rates of conventional batteries, such as the batteries of Comparative Examples 1 and 2, were all relatively larger after storage at 60°C for 50 days. In contrast, the volume expansion rates of the batteries of the examples of the present application could reach 5% (actually, considering measurement errors and the like, the volume expansion rate of 5% was basically a measurable lower limit).

It should be noted that the present application is not limited to the aforementioned embodiments. The above-described embodiments are merely exemplary, and embodiments having substantially the same technical idea and the same effects within the scope of the technical solution of the present application are all included in the technical scope of the present application. Furthermore, without departing from the scope of the subject matter of the present application, various modifications that can come into the mind of those skilled in the art applied to the embodiments and other embodiments constructed by combining partial composition elements of the embodiments are also included in the scope of the present application.

## Claims

1. A secondary battery comprising a positive electrode sheet, a negative electrode sheet, a separator disposed between the positive electrode sheet and the negative electrode sheet, and an electrolyte solution, wherein

    the separator comprises a substrate and an inorganic and/or organic particle layer disposed on at least one surface of the substrate, and
    the separator further comprises an acid scavenging additive located among the inorganic and/or organic particles of the inorganic and/or organic particle layer or located in a form of a separate layer between the substrate and the inorganic and/or organic particle layer,
    the maximum dissolution amount of the acid scavenging additive in the electrolyte solution at 25°C is less than 1% by mass relative to the mass of the electrolyte solution.

2. The secondary battery according to claim 1, wherein the acid scavenging additive comprises at least one selected from a pyridine compound and a phosphine compound,

    the pyridine compound is at least one selected from compounds of chemical formulas I, II and III:

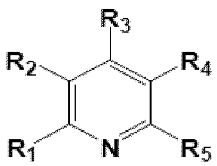
chemical formula I

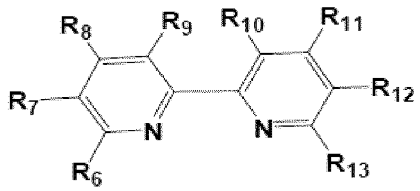
chemical formula II

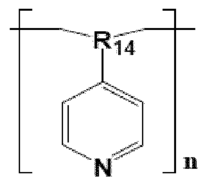
chemical formula III

    the phosphine compound is at least one selected from compounds of chemical formula IV:

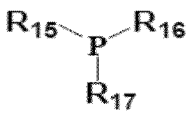
chemical formula IV

    wherein $R_1$ to $R_{13}$ and $R_{15}$ to $R_{17}$ are each independently one selected from the following: alkyl having 1-20 carbon atoms, alkenyl having 2-20 carbon atoms, aryl having 6-26 carbon atoms, aryloxy having 6-26 carbon atoms, and substituted groups thereof formed by substitution with F, Cl, Br, a sulfonic group or sulfonyl, and H, F, Cl and Br; $R_{14}$ is one selected from the following: alkylene having 1-20 carbon atoms, alkenylene having 2-20 carbon atoms, arylene having 7-26 carbon atoms, aryleneoxy having 7-26 carbon atoms, and substituted groups thereof formed by substitution with F, Cl, Br, a sulfonic group or sulfonyl, and n is an integer greater than or equal to 2.

3. The secondary battery according to claim 2, wherein the pyridine compound comprises at least one selected from an isonicotinate, 2-hydroxypyridine-4-carboxylate, 2,2'-bipyridine-4,4'-dicarboxylate, and poly(4-vinylpyridine).

4. The secondary battery according to claim 3, wherein

the isonicotinate comprises at least one selected from lithium isonicotinate, sodium isonicotinate, potassium isonicotinate, aluminum isonicotinate, magnesium isonicotinate, calcium isonicotinate, iron isonicotinate, cobalt isonicotinate, nickel isonicotinate and manganese isonicotinate;
the 2-hydroxypyridine-4-carboxylate comprises at least one selected from lithium 2-hydroxypyridine-4-carboxylate, sodium 2-hydroxypyridine-4-carboxylate, potassium 2-hydroxypyridine-4-carboxylate, aluminum 2-hydroxypyridine-4-carboxylate, magnesium 2-hydroxypyridine-4-carboxylate, calcium 2-hydroxypyridine-4-carboxylate, iron 2-hydroxypyridine-4-carboxylate, cobalt 2-hydroxypyridine-4-carboxylate, nickel 2-hydroxypyridine-4-carboxylate, and manganese 2-hydroxypyridine-4-carboxylate; and
the 2,2'-bipyridine-4,4'-dicarboxylate comprises at least one selected from sodium 2,2'-bipyridine-4,4'-dicarboxylate, potassium 2,2'-bipyridine-4,4'-dicarboxylate, aluminum 2,2'-bipyridine-4,4'-dicarboxylate, magnesium 2,2'-bipyridine-4,4'-dicarboxylate, calcium 2,2'-bipyridine-4,4'-dicarboxylate, iron 2,2'-bipyridine-4,4'-dicarboxylate, cobalt 2,2'-bipyridine-4,4'-dicarboxylate, nickel 2,2'-bipyridine-4,4'-dicarboxylate and manganese 2,2'-bipyridine-4,4'-dicarboxylate.

**5.** The secondary battery according to claim 2, wherein the phosphine compound comprises at least one selected from diethylphenylphosphine, trihexylphosphine, tributylphosphine, 1,6-bis(diphenylphosphino)hexane, tris(o-methoxyphenyl)phosphine, tri-n-octylphosphine, diphenylcyclohexylphosphine, diphenyl-2-pyridinephosphine, 2-(dicyclohexylphosphino)biphenyl, 2-(di-tert-butylphosphino)biphenyl, diphenylethoxyphosphine, tris(2-furyl)phosphine, tricyclopentylphosphine, and diethylphenylphosphine.

**6.** The secondary battery according to any one of claims 1-5, wherein the loading amount of the acid scavenging additive on the separator is 0.01 $g/m^2$-20 $g/m^2$, and optionally 0.1 $g/m^2$-5 $g/m^2$.

**7.** The secondary battery according to any one of claims 1-6, wherein the thickness of the inorganic and/or organic particle layer is 0.1 $\mu$m-10 $\mu$m, and optionally 0.5 $\mu$m-5 $\mu$m.

**8.** The secondary battery according to any one of claims 1-7, wherein the inorganic particle comprises at least one selected from the group consisting of silica, alumina, boehmite, magnesia, titanium dioxide, zinc oxide, and magnesium aluminate oxide; the organic particle comprises at least one selected from the group consisting of polyethylene oxide particles, polyvinylidene chloride particles, polyacrylonitrile particles, polyvinylidene fluoride particles, polymethyl methacrylate particles, and poly(vinylidene fluoride-hexafluoropropylene) copolymer particles.

**9.** The secondary battery according to any one of claims 1-8, wherein the electrolyte solution comprises a solvent and an electrolyte salt, and optionally, the solvent comprises at least one selected from a carbonate solvent, a sulfone solvent, an ether solvent, a sulfonate solvent, a sulfate solvent and a sulfite solvent; and the electrolyte salt comprises a lithium or sodium salt.

**10.** The secondary battery according to any one of claims 1-9, wherein the substrate comprises at least one selected from polyethylene, polypropylene, cellulose and polyimide.

**11.** A battery module comprising the secondary battery according to any one of claims 1-10.

**12.** A battery pack comprising the battery module according to claim 11.

**13.** An electrical apparatus comprising at least one selected from the secondary battery according to any one of claims 1-10, the battery module according to claim 11 or the battery pack according to claim 12.

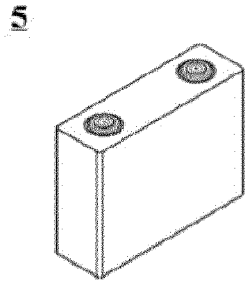


FIG. 1

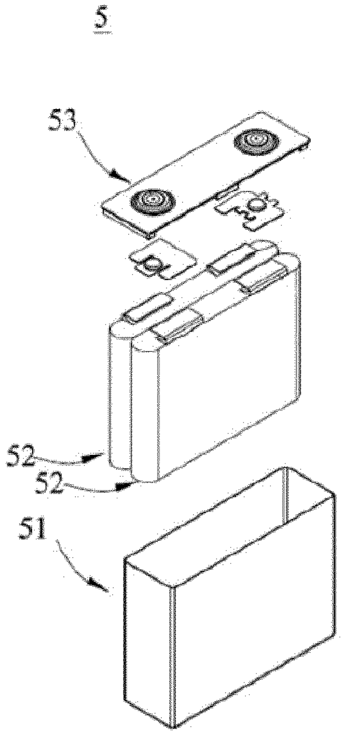


FIG. 2

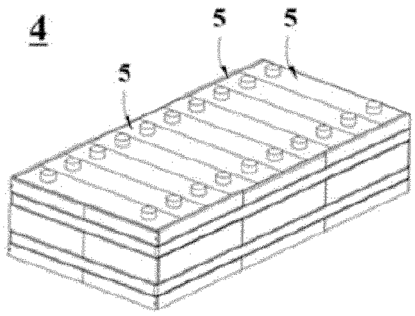


FIG. 3

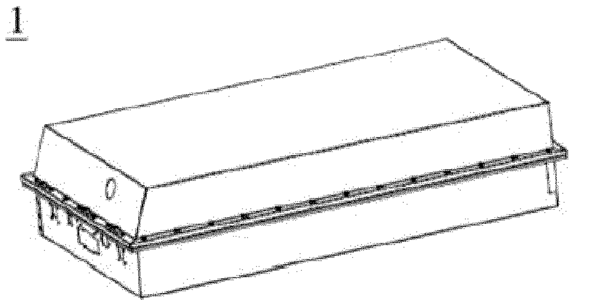


FIG. 4

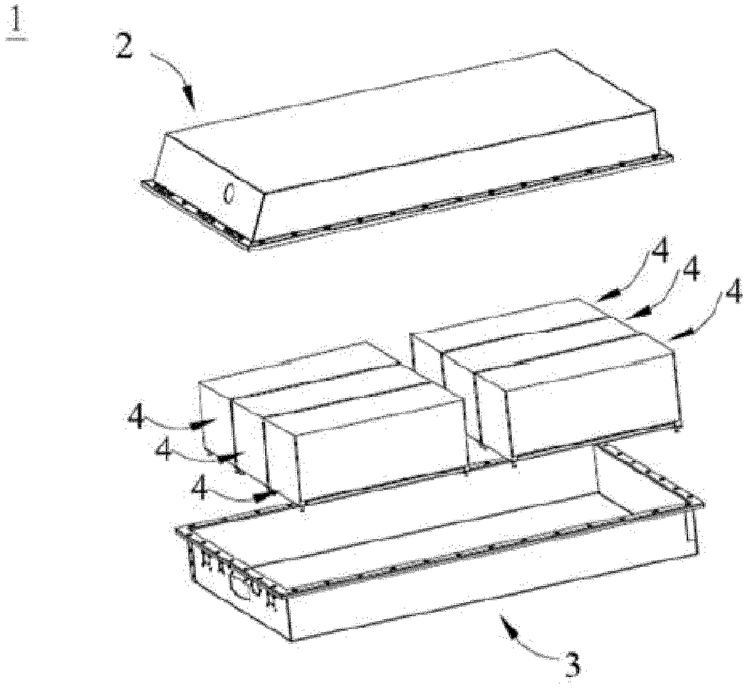


FIG. 5

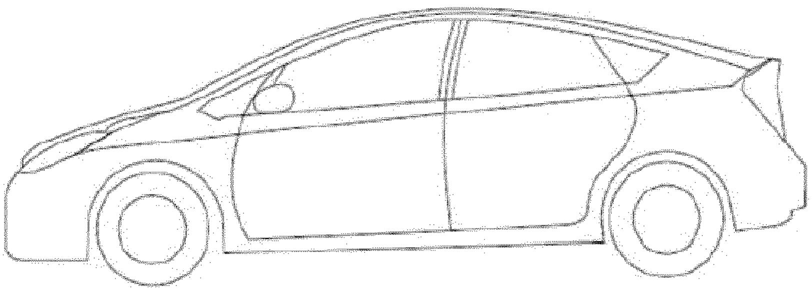

FIG. 6

**INTERNATIONAL SEARCH REPORT**

International application No.
**PCT/CN2021/136599**

**A. CLASSIFICATION OF SUBJECT MATTER**

H01M 10/052(2010.01)i

According to International Patent Classification (IPC) or to both national classification and IPC

**B. FIELDS SEARCHED**

Minimum documentation searched (classification system followed by classification symbols)

H01M

Documentation searched other than minimum documentation to the extent that such documents are included in the fields searched

Electronic data base consulted during the international search (name of data base and, where practicable, search terms used)

CNPAT, CNKI, WPI, EPODOC: 电池, 隔膜, 隔离膜, 隔板, 无机, 有机, 除酸, 添加剂, 副反应, 膦, 异烟酸, 吡啶, 甲酸, 基膦, 电解液, 电解质, battery, separator, inorganic, organic, acid, remov+, additive, reaction, phosph+, isonicotinic, pyridine, formic, electrolyte

**C. DOCUMENTS CONSIDERED TO BE RELEVANT**

| Category* | Citation of document, with indication, where appropriate, of the relevant passages | Relevant to claim No. |
|---|---|---|
| Y | CN 104081576 A (KOKAM CO., LTD.) 01 October 2014 (2014-10-01)<br>description, paragraphs 0013-0038, 0048-0109, and 0128-0139 | 1-13 |
| Y | CN 110994029 A (BEIJING INSTITUTE OF TECHNOLOGY) 10 April 2020 (2020-04-10)<br>description, paragraphs 0004-0034 | 1-13 |
| A | US 2002025477 A1 (MITSUBISHI CHEMICAL CORP.) 28 February 2002 (2002-02-28)<br>entire document | 1-13 |
| A | US 2018138539 A1 (THE REGENTS OF THE UNIVERSITY OF MICHIGAN et al.) 17 May 2018 (2018-05-17)<br>entire document | 1-13 |
| A | CN 103718336 A (LG CHEMICAL LTD.) 09 April 2014 (2014-04-09)<br>entire document | 1-13 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 August 2022** | **08 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/136599**

| Patent document cited in search report | Publication date (day/month/year) | Patent family member(s) | Publication date (day/month/year) |
|---|---|---|---|
| CN 104081576 A | 01 October 2014 | JP 2015505137 A | 16 February 2015 |
| | | KR 20130066746 A | 21 June 2013 |
| | | US 2014370358 A1 | 18 December 2014 |
| | | WO 2013089313 A1 | 20 June 2013 |
| | | EP 2793305 A1 | 22 October 2014 |
| | | KR 101344939 B1 | 27 December 2013 |
| | | EP 2793305 A4 | 23 December 2015 |
| | | JP 6005757 B2 | 12 October 2016 |
| | | CN 104081576 B | 06 March 2018 |
| | | EP 2793305 B1 | 21 March 2018 |
| | | US 10090501 B2 | 02 October 2018 |
| CN 110994029 A | 10 April 2020 | CN 110994029 B | 06 July 2021 |
| US 2002025477 A1 | 28 February 2002 | EP 1172878 A2 | 16 January 2002 |
| | | JP 2002093462 A | 29 March 2002 |
| | | US 6767671 B2 | 27 July 2004 |
| | | EP 1172878 A3 | 25 May 2005 |
| | | EP 1172878 B1 | 22 June 2011 |
| | | JP 4934919 B2 | 23 May 2012 |
| US 2018138539 A1 | 17 May 2018 | WO 2016200870 A1 | 15 December 2016 |
| | | US 11056705 B2 | 06 July 2021 |
| CN 103718336 A | 09 April 2014 | KR 20130022395 A | 06 March 2013 |
| | | WO 2013028046 A2 | 28 February 2013 |
| | | US 2014120402 A1 | 01 May 2014 |
| | | JP 2014524114 A | 18 September 2014 |
| | | EP 2750220 A2 | 02 July 2014 |
| | | WO 2013028046 A3 | 10 May 2013 |
| | | KR 101389022 B1 | 25 April 2014 |
| | | EP 2750220 A4 | 01 April 2015 |
| | | US 9083036 B2 | 14 July 2015 |
| | | JP 5892713 B2 | 23 March 2016 |
| | | EP 2750220 B1 | 22 February 2017 |
| | | CN 103718336 B | 17 May 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105006594 B
- CN 105633467 B
- CN 110970621 B
- CN 1194439 C
- CN 102709591 B